# EUROPEAN PATENT APPLICATION

(11) **EP 4 342 481 A1**
(43) Date of publication of application: **27.03.2024**
(21) Application number: 22382880.7
(22) Date of filing: 26.09.2022
(51) Int. Cl.: A61K 36/07, A61K 9/00, A61P 15/00, A61P 15/02, A61P 31/22, A61P 35/00

(54) **USE OF AN EXTRACT OF HERICIUM ERINACEUS FOR THE TREATMENT OF VAGINAL AND CERVICAL DISEASES**

(71) Applicant: Hifas da Terra SL, 36154 Pontevedra (ES)
(72) Inventor: Fernández de Ana Portela, Catalina, 36154 PONTEVEDRA (ES); Sinde Stompel, Esteban, 36154 PONTEVEDRA (ES); Rodríguez Blanco, Arturo, 36154 PONTEVEDRA (ES); Rubianes Fariña, Diego, 36154 PONTEVEDRA (ES)
(74) Representative: Pons

(57) **Abstract**

The present invention relates to an extract of *Hericium erinaceus,* or the composition comprising same, preferably obtained from the carpophore of said fungus, as well as the method for obtaining said extract by infusion and percolation. Furthermore, the present invention also relates to the extract of *Hericium erinaceus* for use in the treatment and/or prevention of vaginal or cervical epithelial diseases and lesions, where said diseases are caused by an infectious agent, in particular by the human papillomavirus (HPV).

## Description

The present invention belongs to the field of medicine, specifically to the treatment of vaginal and cervical diseases and/or lesions. The present invention relates to an extract of *Hericium erinaceus,* preferably obtained from the carpophore of said fungus, for use in the treatment and/or prevention of vaginal or cervical epithelial diseases and lesions, caused by an infectious agent, in particular by the human papillomavirus (HPV).

### STATE OF THE ART

Vaginal infections are considered the most common gynaecological problem in women of reproductive age, affecting millions of people each year and being the most common cause of gynaecological medical care. The most common infections include bacterial vaginosis, aerobic vaginitis, vulvovaginal candidiasis, trichomoniasis and viral infections. Viral infections are the most difficult to treat. In particular, human papillomavirus (HPV) infection is among the most common sexually transmitted diseases in the world, with the highest prevalence among women under 25 years of age. HPV infection is the leading cause of cervical cancer and is linked to many other types of cancer.

The human papillomavirus (HPV) is an infectious agent belonging to the virus family *Papillomaviridae,* the members of which exhibit tropism for the cutaneous epithelium and the mucosal epithelium.

There are more than 100 subtypes of HPV, of which 13 are considered high risk. The HPV-16 and HPV-18 subtypes are the most oncogenic and prevalent, accounting for about 70% of cervical cancer cases. It is estimated that 80% of sexually active women will have been infected at some point. More than 90% of HPV infections are transient and cleared by an immune response within 6 to 18 months, although a new HPV reinfection may occur. The persistence of the virus can lead to the development of low-grade squamous intraepithelial lesions (LSIL) in the cervix. LSILs are characterised by the presence of abnormal cells on the surface of the cervix. LSILs can lead to high-grade squamous intraepithelial lesions (HSILs) that can ultimately lead to cervical cancer. Factors that correlate with higher persistence rates include immunodeficiency. Incidence data indicate that cervical cancer is the second most common tumour in women worldwide. It is estimated that each year there are more than 500,000 new cases of cervical cancer and approximately 280,000 deaths.

There are vaccines for the treatment of HPV but they are not effective against all serotypes. Furthermore, when a person has already been exposed to the virus before vaccination, the efficacy of the vaccine against serotypes such as HPV-16 or HPV-18 decreases.

Despite the large number of successfully vaccinated women around the world, many populations still have not had the opportunity to be vaccinated. In particular, current HPV immunisation programmes target only 12% of young adolescent women worldwide, while the remaining women will miss the opportunity to get vaccinated without rapid implementation of the HPV vaccine. Many countries in Africa and Asia, which represent the majority of the world's population, have very vulnerable populations at a very high risk of developing cervical cancer and other HPV-related diseases.

Patent document WO2015136096A1 discloses a topical composition comprising an extract of *Coriolus versicolor for* the prevention and/or treatment of vaginal or cervical disorders caused by different infectious agents, including the human papillomavirus, among others.

Therefore, it is necessary to provide alternative strategies to combat HPV infection and cervical cancer, particularly in the early stages of HPV infection.

### DESCRIPTION OF THE INVENTION

The present invention relates to an extract of *Hericium erinaceus,* preferably obtained from the carpophore of said fungus, for use in the treatment and/or prevention of vaginal or cervical epithelial diseases and lesions, preferably caused by an infectious agent, in particular by the human papillomavirus (HPV).

The inventors have obtained greater *in vitro* therapeutic activity in HeLa cell lines with the extract of *Hericium erinaceus* than with extracts derived from other species of fungi such as, for example, *Lentinula edodes, Coriolus versicolor, Grifola frondosa or Ganoderma lucidum* (see Table 1 of the examples). Furthermore, the inventors have discovered that with the carpophore extract of *Hericium erinaceus* a surprisingly higher percentage inhibition of HeLa cells is obtained than when a mycelium extract of this fungus is used (see Table 2 of the examples). By way of example, when using a carpophore extract at concentrations of 1 mg/mL, 0.1 mg/mL and 0.02 mg/mL, a percentage inhibition of HeLa cells of 42%, 46% and 41%, respectively, is obtained, while the use of a mycelial extract of *Hericium erinaceus,* at a concentration of 0.01 mg/mL, gives an inhibition of between 1 and 8%.

Therefore, in a first aspect, the present invention relates to an extract of *Hericium erinaceus* obtained from the carpophore, hereinafter "the extract of the invention" or "the carpophore extract of the invention".

*Hericium erinaceus* is a basidiomycete of the family *Hericiaceae,* considered a natural probiotic that helps to restore microbiota and contains a large number of bioactive compounds, especially β-glucan polysaccharides, which are responsible for the anti-cancer, immunomodulatory, antioxidant, neurostimulatory and neuroprotective activities of this species.

The term "extract of *Hericium erinaceus"* refers to one or more compounds, one or more substances, a sample, concentrate or isolated product obtained, derived or extracted from the fungus *Hericium erinaceus.* This includes complete extracts of *H. erinaceus,* but also pure or semi-pure preparations of biologically active compounds obtained from *H. erinaceus,* in particular obtained from the carpophore of *H. erinaceus,* as well as the powder of the carpophore. The extract of *Hericium erinaceus* can be obtained through physical and/or chemical extraction methods. The extract of the invention also includes, preferably, combinations of other known components and/or active compounds that combine to substantially mimic the composition and/or activity of an extract of *H. erinaceus* of natural origin.

The term "carpophore" refers to the reproductive part of the fungus, also called fruiting body, sporocarp or mushroom.

Examples of methods for obtaining the extract of the invention include, but are not limited to, pressing, maceration, maceration with stirring, ultrasound extraction, percolation or repercolation, steam distillation, hydrodistillation, decantation, filtration, evaporation and/or chromatography. Preferably, the extraction method of the invention is percolation.

In a particular embodiment, the extract of the invention is obtained by a method comprising the following steps:
(i) mixing the entire carpophore or portions of carpophore of *Hericium erinaceus,* preferably smaller than 5 cm, with an aqueous solution and/or alcohol, preferably in a solid:liquid ratio of 1:5 to 1:40,
(ii) subjecting the heterogeneous mixture obtained in (i) to a temperature between 20°C and 70°C for between 30 and 60 minutes, obtaining an infusion of the mixture obtained in (i),
(iii) percolating or filtering the infusion obtained in (ii), obtaining the carpophore extract in the liquid fraction or solution.

The extract of the invention can be obtained from the entire carpophore or carpophore cut into small pieces or portions smaller than 5 centimetres (cm), preferably smaller than 4 cm, more preferably smaller than 3 cm or smaller than 2 cm.

In another particular embodiment, the extract of the invention is obtained from carpophore portions smaller than 1 cm.

The entire carpophore or the carpophore pieces are mixed with a solvent, preferably where the solvent is selected from the list consisting of: an aqueous solution (more preferably water), an alcohol (more preferably ethanol, methanol, butanol, 2-propanol or pentanol), propane, butane, ethyl acetate, carbon dioxide, acetone, nitrous oxide or a mixture thereof.

In another more particular embodiment, the extract of the invention is obtained by mixing the entire carpophore or the portions of carpophore with a solvent selected from the list consisting of: an aqueous solution (more preferably water), an alcohol (more preferably ethanol, methanol, butanol, 2-propanol or pentanol), propane, butane, ethyl acetate, carbon dioxide, acetone, nitrous oxide or a mixture thereof.

In another more particular embodiment, the extract of the invention is obtained by mixing the entire carpophore or the portions of carpophore with aqueous solution and alcohol, preferably water and/or alcohol.

In another even more particular embodiment, the extract of the invention is obtained by mixing the entire carpophore or the portions of carpophore with water and/or ethanol, more preferably with a mixture of water and ethanol.

When a mixture of ethanol and water is used, the ethanol is preferably found in a ratio between 10% by volume and 60% by volume (where the water would be found in a ratio between 90% and 40% by volume, respectively), more preferably between 20% and 60% by volume (where the water would be found in a ratio between 80% and 40% by volume, respectively), even more preferably between 40% and 60% by volume (where the water would be found in a ratio between 60% and 40% by volume, respectively).

In another more particular embodiment, the extract of the invention is obtained by mixing the entire carpophore or the portions of carpophore with water and ethanol in a water:ethanol ratio of 50% and 50% by volume, in other words, in a water:ethanol ratio of 1:1.

Particularly, to obtain the extract of the invention, the ratio of the entire carpophore or the portions of carpophore (solid) in the solvent (liquid), in other words, the solid:liquid ratio, is from 1:5 to 1:40.

In another particular embodiment, the ratio of the entire carpophore or the portions of carpophore (solid) in the solvent (liquid) is from 1:10 to 1 :30, preferably 1:20.

The time required for extraction is usually adapted to the starting fungal material, the extraction method, the extraction temperature, the ratio between the solvent and the fungal material and the like.

Once the mixture of the entire carpophore or the portions of the carpophore and the solvent has been obtained, said mixture is subjected to a temperature of between 20 and 70°C for between 30 and 60 minutes (step (ii)), obtaining an infusion of the mixture obtained.

In another more particular embodiment, the mixture of the entire carpophore or the portions of carpophore and the solvent is subjected to a temperature of between 30 and 60°C, preferably between 35 and 55°C, for between 35 and 55 minutes, preferably between 40 and 50 minutes.

In another more particular embodiment, the mixture of the entire carpophore or the portions of carpophore and the solvent is subjected to a temperature of 36, 37, 38, 39, 40, 41, 42, 43, 44, 45, 46, 47, 48, 49, 50, 51, 52, 53 or 54°C for 41, 42, 43, 44, 45, 46, 47, 48 or 49 minutes.

In another even more preferred embodiment, the mixture of the entire carpophore or the portions of carpophore and the solvent is subjected to a temperature of 50°C for 45 minutes.

Optionally, the extraction step may be repeated on the same solid under the same conditions described above, in other words, step (ii) may optionally be repeated at least once.

After extraction (step ii), the mixture of solvent and extracted compounds that forms after the solvent comes into contact with the fungal material is separated from the fungal material by means of solid-liquid filtration by methods such as percolation, obtaining the carpophore extract of the invention in the liquid fraction.

The term "percolation" refers to the action of a liquid to penetrate, pass or move through the pores of some body or environment, in other words, the slow passage of fluids through porous materials. Synonyms of this term in the state of the art are filtration and leaching processes.

The bioactive compounds present in the extract, mainly phenolic compounds, usually have a limited useful life against oxidation. Therefore, additional drying of the extract (in the liquid fraction) can be optionally applied to prolong its useful life. One of the methods that can be applied for drying bioactive extracts is spray drying.

Thus, in another more preferred embodiment, the method for obtaining the extract of the invention further comprises a step (iv) of spray drying the solution obtained in (iii) at a temperature between 135 and 195°C.

In another even more particular embodiment, the temperature of step (iv) is between 145 and 185°C, preferably between 165 and 175°C.

During spray drying, an encapsulating agent can be added, for example, maltodextrin.

Thus, in another more preferred embodiment, the method for obtaining the extract of the invention further comprises a step (v) of adding an encapsulating agent, preferably the encapsulating agent is found in a ratio of between 1% m/m and 50% m/m (where the extract would be found in a ratio between 99% and 50% m/m, respectively), more preferably between 20% and 40% m/m (where the extract would be found in a ratio between 80% and 60% m/m, respectively), even more preferably between 25% and 35% m/m (where the extract would be found in a ratio between 75% and 65% m/m, respectively).

In another more particular embodiment, the extractencapsulating agent ratio is 71.4% m/m and 28.57% m/m, in other words, in an extract:encapsulating agent ratio of 4:14.

The term "encapsulating agent" refers to a substance and/or substances that prevent agglomeration, caking and stickiness of the ingredient that is subjected to spray drying, increasing its stability and improving its bioavailability.

Examples of encapsulating agents include, but are not limited to, maltodextrin, starch and derivatives, corn syrups, saccharose, cyclodextrins, carboxymethylcellulose, methylcellulose, ethylcellulose, nitrocellulose, acetylcellulose, gum arabic, sodium alginate, carrageenan, mono- and diglycerides, whey, zein, gluten and/or casein.

In another more preferred embodiment, the method for obtaining the extract of the invention further comprises a step (v) of adding an encapsulating agent, where the encapsulating agent is maltodextrin.

The carpophore extract of *Hericium erinaceus* to which the present invention relates comprises, preferably, 24.37% m/m of beta-glucans, 51.2 µg/g of GABA, 348.2 µg/g of ergosterol and 0.2% m/m of Hericenone C.

As understood by a person skilled in the art, the extract of the invention can be part of a composition. Thus, another aspect of the invention relates to a composition, hereinafter "the composition of the invention", which comprises the extract of *Hericium erinaceus* obtained from carpophore of the invention.

The composition of the invention can be formulated in solid, semi-solid, gel, liquid or gaseous forms, such as a tablet, capsule, caplet, powder, granule, ointment, solution, suppository, injection, inhalant, gel, microsphere or aerosol.

In another preferred embodiment of the invention, the composition of the invention is in liquid, gel or solid form.

In another preferred embodiment of the invention, the composition of the invention is formulated in solid form, more preferably in capsule format.

The composition of the invention can be presented in any form of administration. For example, it may be in a form adapted for oral, topical vaginal, parenteral, rectal or cervical administration.

Thus, in another preferred embodiment of the invention, the composition is formulated for its oral, topical vaginal, parenteral, rectal or cervical administration.

Particularly, in another more preferred embodiment of the invention, the composition is administered simultaneously orally and topical vaginally.

The composition of the invention can be formulated (i) for food administration, alone or as part of the food consumed in the subject's diet or (ii) for pharmaceutical administration, alone or as part of pharmaceutical products that are administered to the subject by any means of administration.

Thus, in another preferred embodiment of the composition of the invention, the composition of the invention is a pharmaceutical composition or a nutritional or food composition, hereinafter the "pharmaceutical composition of the invention" and the "nutritional composition of the invention", respectively.

The term "pharmaceutical composition" refers to a set, mixture, combination of components or substances comprising the extract of the invention in any concentration, and which improves the subject's state of health or reduces the risk of disease. Said pharmaceutical composition can be a medicine. However, the term "pharmaceutical composition" also includes medical devices, provided that the "medical device" is intended to be used on subjects, preferably people, separately or in combination with other products, for any of the following specific medical purposes: prevention, treatment or relief of a disease or treatment, alleviation or compensation of a lesion or disability, and its main mechanism of action is not pharmacological, metabolic or immunological.

The extract of the invention can be administered to the subject in a therapeutically effective dose, which can vary over a wide range. In the present invention, the expressions "therapeutically effective dose" or "therapeutically effective amount", used interchangeably herein, refer to the amount or dose of extract of the invention that, when administered to a subject, preferably a mammal, and more preferably a human, is sufficient to prevent and/or treat vaginal and cervical diseases and lesions. The therapeutically effective amount will vary, for example, based on the metabolic stability and duration of the action of the extract; age, body weight, general state of health, gender and diet of the subject; the mode and time of administration; the excretion rate, the combination with other drugs; the severity of the particular disease or pathological condition of said subject. It is routine practice for a person skilled in the art, for example, a specialist doctor, to determine the therapeutically effective dose for each subject, preferably a human, according to their own knowledge and the physiological conditions or variables of the subject.

In another preferred embodiment, the composition comprises the extract of *H*. *erinaceus* of the invention in a concentration comprised between 0.005% and 25%, in %m/m, depending on the form in which said composition is presented, for example, depending on whether the composition is a topical composition or an oral composition, or whether it is in liquid, gel or solid form.

The term "%m/m" refers to the mass percentage of the extract, in other words, a measure of the concentration that indicates the mass of solute (extract of the invention) per 100 mass units of the solution.

In another even more preferred embodiment of the invention, the composition, preferably when it is a topical composition, comprises the extract of *Hericium erinaceus* of the invention in a concentration between 0.5% and 5% m/m.

In another even more preferred embodiment of the invention, the topical composition comprises the extract of *Hericium erinaceus* of the invention in a concentration between 0.5% and 3% m/m, preferably in a concentration of 1%, 1.5%, 2% or 2.5% m/m, more preferably 1% m/m.

In another preferred embodiment, the composition, preferably when it is an oral composition, comprises the extract of *Hericium erinaceus* of the invention in a concentration of between 1 and 25% m/m, preferably between 5 and 15% m/m, more preferably 5, 6, 7, 8, 9, 10, 11, 12, 13, 14 or 15% m/m.

In another preferred embodiment of the invention, the pharmaceutical composition further comprises a pharmaceutically acceptable carrier and/or excipient.

The term "excipient" refers to a substance that aids the absorption of any of the components of the pharmaceutical composition, stabilises said components, modifies their organoleptic properties or determines the physico-chemical properties of the pharmaceutical composition and its bioavailability. Thus, the excipients can have the function of binding the components (for example, starches, sugars or celluloses), sweetening, colouring, protecting the active ingredient (for example, to isolate it from air and/or moisture), filling a pill, capsule or any other presentation, or a disintegrating function to facilitate the dissolution of the components, without excluding other excipients not listed herein. Therefore, the term "excipient" is defined as a material that, included in galenic forms, is added to active ingredients or to their associations to allow for their preparation and stability, to modify their organoleptic properties or to determine the physico-chemical properties of the pharmaceutical composition and its bioavailability. Examples of excipients include, but are not limited to: organic rice extract, rice fibre, rice protein, silicon dioxide, calcium carbonate, dicalcium phosphate, sorbitol, sucralose, xylitol, blueberry aroma, citric acid, lactic acid, sodium benzoate, sorbic acid or a combination thereof.

The term "vehicle" or "carrier" is a substance used in the drug to dilute any of the components or compounds of the composition to a certain volume or weight; or that even without diluting these components or compounds is capable of allowing for better dosage and administration and/or giving consistency and shape to the drug. Examples of pharmaceutically acceptable carriers or vehicles include, without limitation, water, saline solutions, alcohol, vegetable oils, polyethylene glycols, gelatin, lactose, starch, amylose, magnesium stearate, talc, surfactants, salicylic acid, viscous paraffin, perfume oil, monoglycerides and diglycerides of fatty acids, petroethral fatty acid esters, hydroxymethylcellulose, hydroxypropyl methylcellulose, microcrystalline cellulose, polyvinylpyrrolidone, chitosan and a combination thereof. Preferably, the carrier is chitosan, water or hydroxypropyl methylcellulose.

Furthermore, the excipient and the carrier must be "pharmacologically acceptable", in other words, the excipient and the carrier are permitted and evaluated to not cause harm to the subject to whom it is administered, in other words, to be compatible with the extract of the invention.

Thus, in another preferred embodiment, the pharmaceutically acceptable carrier and/or excipient of the composition of the invention is water, chitosan, hydroxypropyl methylcellulose, lactic acid, sodium hyaluronate, blueberry aroma, citric acid, sorbic acid, sodium benzoate, organic rice extract, rice fibre, rice protein, silicon dioxide, calcium carbonate, dicalcium phosphate, sorbitol, sucralose, xylitol and/or combinations thereof.

In another more preferred embodiment, the pharmaceutically acceptable carrier and/or excipient of the composition of the invention, preferably when it is an oral composition, is organic rice extract, rice fibre, rice protein, silicon dioxide, calcium carbonate, dicalcium phosphate, sorbitol, sucralose, xylitol, hydroxypropyl methylcellulose and/or combinations thereof.

In another more preferred embodiment, the pharmaceutically acceptable carrier and/or excipient of the composition of the invention, preferably when it is a topical composition, is lactic acid, sodium hyaluronate, blueberry aroma, citric acid, sorbic acid, sodium benzoate, chitosan, water and/or combinations thereof.

As understood by a person skilled in the art, the composition of the invention may comprise prebiotics, probiotics, immunomodulatory agents, tissue regenerating agents, moisturising agents, antiseptic agents, fungus or fungus extract, vitamins and/or minerals.

Thus, in another preferred embodiment, the composition further comprises at least one prebiotic, probiotic, immunomodulatory agent, tissue regenerating agent, moisturising agent, antiseptic agent, fungus or fungus extract, vitamin, mineral and/or a combination thereof.

The term "probiotic" as used in the present invention refers to a composition comprising microorganisms or a microorganism which, when supplied in adequate amounts, have beneficial effects on the health of the recipient subject or organism. Examples of probiotics that can be comprised in the composition of the invention include, but are not limited to, *Saccharomyces cerevisae, Lactobacillus rhamnosus, Lactobacillus gasseri, Lactobacillus plantarum, Lactobacillus paracasei, Lactobacillus acidophilus, and Lactobacillus crispatus.*

The term "prebiotic" as used in the present invention refers to substances or compounds (mainly consisting of non-starch polysaccharides and oligosaccharides) that nourish selected groups of microorganisms by promoting the growth of bacteria or other beneficial microorganisms against other bacteria or microorganisms. Examples of prebiotics that can be comprised in the composition of the invention include, but are not limited to, an alpha-glucan oligosaccharide, inulin and/or fructooligosaccharides. Alpha-glucans are polysaccharides made up of D-glucose monomers linked together by glycosidic bonds in alpha configuration. Fructooligosaccharides (FOS) and inulin are a type of soluble fibre made up of fructose units linked together by means of glycosidic bonds in beta configuration and they may contain an initial glucose molecule. Alpha-glucans are commercially available. The effective amount of alpha-glucan is comprised between 0.001 and 5% m/m, preferably between 0.01 and 2% m/m, more preferably 1.5% m/m.

Furthermore, the composition of the invention may comprise an immunomodulatory agent. The term "immunomodulatory agent" as used in the present invention refers to substances or compounds that have an effect on the immune system.

In another preferred embodiment of the invention, the immunomodulatory agent is a beta-glucan. β-glucans are natural polysaccharides made up of D-glucose units linked with a β-glycosidic bond. Beta-glucans have been described to have a variety of biological activities, with their immunomodulatory activity being noteworthy. The immunomodulatory activity of β-glucans is based on the conformational structure. β-glucans can be obtained from various sources such as grains, fungi and yeasts.

In another more preferred embodiment of the invention, beta-glucan is a carboxylated beta-glucan. In another embodiment, carboxylated beta-glucan is a derivative of β-(1,3) and β-(1,6) glucan, a natural polysaccharide present in yeast. In addition to immunomodulatory activity, various commercial carboxylated beta-glucans obtained from yeast have skin protective, regenerating, moisturising and re-epithelialising properties. The effective amount of beta-glucan is comprised between 0.001 and 5% m/m, preferably between 0.01 and 1% m/m, more preferably 0.5% m/m.

Furthermore, the composition of the invention may comprise a tissue regenerating agent. "Tissue regenerating agent" is understood to be a compound that can promote skin repair, re-epithelialisation and/or wound healing. Examples of tissue regenerating agents in the composition of the invention include, but are not limited to, extract of *Centella asiatica* and allantoin.

In another preferred embodiment of the invention, the tissue regenerating agent is *Centella asiatica. Centella asiatica* has been used as a traditional medicine in Malaysia and other parts of Asia for hundreds of years. It has been used for various medicinal purposes, especially wound healing, cell repair and renewal, providing firmness and elasticity to the skin, and antioxidant action, although its main application has been to promote wound healing.

The term "extract of *Centella asiatica"* refers to an extract obtained from *Centella asiatica* which contains a high concentration of biologically active compounds, mainly triterpenoids. The triterpenoids contained in these extracts are mainly asiatic acid, madecassic acid and asiaticoside. "Extract of *Centella asiatica"* as used in the present invention includes any of the available extracts of *Centella asiatica,* as well as purified or semi-purified preparations of biologically active compounds of *Centella asiatica* or a particular biologically active compound obtained from *Centella asiatica.* Examples of commercial extracts of *Centella asiatica* are standardised extracts in madecassic acid and asiaticoside, in particular an extract standardised to 50-70% m/m of madecassic acid and 10-20% m/m of asiaticoside.

In another more preferred embodiment, the extract of *Centella asiatica* comprises between 50 and 70% m/m of madecassic acid and between 10 and 20% m/m of asiaticoside. The effective amount of *Centella asiatica* is comprised between 0.001 and 5% m/m, preferably between 0.01 and 2% m/m, more preferably 1% m/m.

In another preferred embodiment of the invention, the tissue regenerating agent is allantoin. Allantoin is a heterocyclic organic compound. It has skin protective, conditioning, soothing and regenerating action. The effective amount of allantoin is comprised between 0.001 and 5% m/m, preferably between 0.01 and 1% m/m, more preferably 0.5% m/m.

Furthermore, the composition of the invention may comprise a moisturising agent. "Moisturising agent" is understood to be a compound that can promote tissue hydration and/or provide elasticity.

In another preferred embodiment of the invention, the moisturising agent is hyaluronic acid.

Hyaluronic acid (HA) is a naturally occurring linear polysaccharide composed of repeating units of D-glucuronic acid and N-acetyl-d-glucosamine disaccharide that has numerous functions. Among the properties of hyaluronic acid, hydration and viscoelastic properties are worth noting. An example of hyaluronic acid is sodium hyaluronate. The effective amount of hyaluronic acid is comprised between 0.001 and 5% m/m, preferably between 0.01 and 2% m/m, more preferably 0.5% m/m.

Furthermore, the composition of the invention may comprise an antiseptic agent.

"Antiseptic agent" is understood to be a compound that has an inhibitory action on fungi, bacteria and/or viruses, such as Aloe vera leaf juice.

In another preferred embodiment of the invention, the antiseptic agent is Aloe vera leaf juice. The Aloe vera plant has been known and used for centuries for its health, beauty, medicinal and skin care properties. The botanical name of Aloe vera is *Aloe barbadensis.* It belongs to the family *Asphodelaceae* (*Liliaceae*), and mainly grows in the dry regions of Africa, Asia, Europe and the Americas. The active ingredients of Aloe vera, mainly glucomannan, a polysaccharide rich in mannose, and gibberellin, a growth hormone, interact with growth factor receptors on the fibroblast, thus stimulating its activity and proliferation, which in turn significantly increases collagen synthesis after topical and oral administration of Aloe vera. Aloe vera has epithelialising and antiseptic effects, among others. Aloe vera contains 6 antiseptic agents: lupeol, salicylic acid, ureic nitrogen, cinnamic acid, phenols and sulphur. All have inhibitory action on fungi, bacteria and viruses. The effective amount of *Aloe barbadensis* is comprised between 0.001 and 5% m/m, preferably between 0.01 and 2% m/m, more preferably 1.5% m/m.

In another preferred embodiment of the invention, the composition of the invention further comprises *Aloe barbadensis* juice, alpha-glucan oligosaccharide, inulin, fructooligosaccharides, hyaluronic acid, beta-glucan, *Centella asiatica,* allantoin, *Cordyceps sinensis, Lentinula edodes, Ganoderma lucidum, Saccharomyces cerevisae, Lactobacillus rhamnosus, Lactobacillus gasseri, Lactobacillus plantarum, Lactobacillus paracasei, Lactobacillus acidophilus, Lactobacillus crispatus,* vitamin C, vitamin D, vitamin B12, niacin, folic acid, vitamin E, vitamin A, selenium, zinc and/or a combination thereof.

In another preferred embodiment of the invention, the composition, when it is a topical composition, comprises an alpha-glucan oligosaccharide, a carboxylated beta-glucan, an extract of *Centella asiatica*, allantoin, hyaluronic acid, and *Aloe barbadensis* juice.

In another preferred embodiment of the invention, the composition, when it is a topical composition, includes water, chitosan, lactic acid, *Aloe barbadensis* leaf juice, alpha-glucan oligosaccharide, hyaluronic acid, extract of *Centella asiatica,* allantoin, blueberry aroma, citric acid, lactic acid and sodium benzoate.

In another even more preferred embodiment of the invention, the composition further comprises at least one fungus or fungus extract, vitamin, probiotic and/or mineral, preferably when the composition is an oral composition.

In another even more preferred embodiment of the composition of the invention, preferably the oral composition, it further comprises a fungus extract, wherein the fungus is *Cordyceps sinensis, Lentinula edodes* and/or *Ganoderma lucidum.* The effective amount of extract of *Cordyceps sinensis,* extract of *Lentinula edodes* and extract of *Ganoderma lucidum* is comprised between 1 and 25% m/m, preferably between 5 and 15% m/m, preferably 5, 10, or 15% m/m of each species.

In another even more preferred embodiment of the composition of the invention, preferably the oral composition, it further comprises vitamin C, vitamin D, vitamin E, vitamin A, vitamin B12, niacin and/or folic acid. The effective amount of vitamin C is comprised between 1 and 15% m/m, preferably 7.5% m/m. The effective amount of vitamin D is comprised between 0.01 and 5% m/m, preferably between 0.1 and 1% m/m, more preferably 0.2% m/m. The effective amount of vitamin E is comprised between 0.01 and 5% m/m, preferably between 0.1 and 4% m/m, preferably 2% m/m.

In another even more preferred embodiment of the composition of the invention, preferably the oral composition, it further comprises a probiotic that is selected from the list consisting of: *Saccharomyces cerevisae, Lactobacillus rhamnosus, Lactobacillus gasseri, Lactobacillus plantarum, Lactobacillus paracasei, Lactobacillus acidophilus and Lactobacillus crispatus.* The effective amount of the probiotic may be comprised between 1 and 10% m/m, for example, 8% m/m.

In another even more preferred embodiment of the composition of the invention, preferably the oral composition, the mineral is selenium and/or zinc. The effective amount of zinc is comprised between 0.001 and 5% m/m, preferably between 0.01 and 1% m/m, more preferably 0.5% m/m. The effective amount of selenium may be comprised between 0.001 and 5% m/m, preferably between 0.01 and 1% m/m, more preferably 0.6% m/m.

In another even more preferred embodiment, the oral composition of the invention comprises *Cordyceps sinensis, Lentinula edodes, Ganoderma lucidum, Saccharomyces cerevisae, Lactobacillus rhamnosus, Lactobacillus gasseri, Lactobacillus plantarum, Lactobacillus paracasei, Lactobacillus acidophilus, Lactobacillus crispatus,* vitamin C, vitamin D, vitamin B12, niacin, folic acid, vitamin E, vitamin A, selenium, zinc and/or a combination thereof.

As described above, the composition of the invention may be a nutritional composition.

The term "nutritional composition" of the present invention refers to a food that, regardless of providing nutrients to the subject who takes it, beneficially affects one or more functions of the body, in a way that provides a better state of health and well-being.

In the event that the composition of the invention is formulated as a nutritional composition, said nutritional composition may be a food or incorporated into a food or food product intended for human consumption.

Thus, in a particular embodiment, the nutritional composition of the invention is a food or a nutritional supplement.

The term "supplement", synonymous with any of the terms "dietary supplement", "nutritional supplement", or "food supplement", refers to products or preparations whose purpose is to supplement the normal diet of a subject and which consist of concentrated nutrient sources or other substances with a nutritional or physiological effect.

The nutritional supplement can be presented in simple or combined form and marketed in dosage form, in other words, in capsules, caplets, tablets and other similar forms, powder sachets, liquid ampoules and dropper bottles and other similar forms of liquids and powders designed to be taken in a single quantity.

Examples of foods that may comprise the first nutritional composition of the invention include, but are not limited, dairy products and beverages. Examples of dairy products include, but are not limited to, products derived from fermented milk (for example, but not limited to, yoghurt) or unfermented milk (for example, but not limited to, whey).

As described above, the extract of *Hericium erinaceus* obtained from carpophore can be used as medicine.

Thus, another inventive aspect relates to the extract obtained from carpophore and to the composition of the invention for use as a medicament, the "first medical use" of the invention.

The "medicament" is any substance or combination of substances that has properties for treating or preventing diseases in humans or that can be used in humans or administered to humans for the purpose of restoring, correcting or modifying physiological functions exerting a pharmacological, immune or metabolic effect, or establishing a medical diagnosis. In particular, it implies a preventive or therapeutic effect on vaginal or cervical epithelial diseases and lesions, such as cervical cancer, and preferably those caused by an infectious agent (for example, HPV), and involves elimination of the HPV infection.

The present invention relates to the use of the extract of *Hericium erinaceus* obtained from carpophore (the extract of the invention) for the preparation of a medicament.

As described above, both the extract of *Hericium erinaceus* obtained from carpophore (the extract of the invention) and the extract obtained from mycelium, as well as the compositions comprising them respectively, can be used to prevent and reverse symptoms of vaginal or cervical diseases and lesions such as, for example, those caused by an infectious agent.

Thus, another aspect of the invention relates to an extract of *Hericium erinaceus,* obtained from carpophore (extract of the invention) or from mycelium, or a composition comprising it for use in the treatment and/or prevention of vaginal or cervical diseases and/or lesions, preferably vaginal or cervical epithelial lesions. In a preferred embodiment, such diseases and/or lesions are caused by an infectious agent. Hereinafter the "second medical use".

In a more preferred embodiment, the extract of *Hericium erinaceus* is obtained from carpophore (the extract or the composition of the invention).

The extract of *Hericium erinaceus* can be mycelial or from carpophore. The mycelial extract relates to the extract obtained, preferably, by the following production method, comprising the following steps:
(a) culturing a strain of *Hericium erinaceus* under aerobic conditions, in a liquid culture medium comprising brown sugar, potato flour, carrot flour, rice protein, oatmeal and virgin olive oil, to obtain a fermented product, and
(b) extracting, filtering and/or concentrating the fermented product obtained in step (a) to obtain a mycelial extract.

In a more particular embodiment, the cultivation of step a) is carried out between 4 and 6 days (both included), at a temperature between 25° and 28°C (both included), and at a pH of 6.

In another particular embodiment of the method for producing mycelium extract, the extraction in step b) is carried out by applying pressure and temperature, between 0.5 and 1.5 bar and 115 and 125°C, for 8 to 12 minutes (including the ends of all ranges). Preferably, step b) is carried out at 1 bar and 121°C, for 9 to 11 minutes (including both ends).

After extraction, filtration of the resulting product is carried out. Thus, in another particular embodiment of the method for obtaining the mycelial extract, filtration is carried out through a 190 µm filter and subsequently through a 22 µm filter to remove residual biomass.

Extraction by pressure and temperature allow the product to be sterilised and therefore stabilised.

Alternatively, the present invention relates to the use of the extract of *Hericium erinaceus,* obtained from carpophore or from mycelium, for the preparation of a medicine for the treatment and/or prevention of vaginal or cervical diseases and/or lesions, preferably caused by an infectious agent.

The term "mycelium" refers to the group of hyphae that form the vegetative part of a fungus. As far as the present invention is concerned, the mycelium of *Hericium erinaceus* is not very dense, and is white and cottony.

The term "treatment", as understood in the present invention, refers to combating the effects caused by a disease, in particular vaginal or cervical diseases or lesions, preferably caused by an infectious agent, in a subject (preferably a human), including:
(i) inhibiting the disease or pathological condition, in other words, stopping its development;
(ii) alleviating the disease or pathological condition, in other words, causing the remittance of the disease or pathological condition or the symptoms thereof;
(iii) stabilising the disease or pathological condition.

The term "prevention" as understood in the present invention consists of preventing the onset of a disease, specifically vaginal or cervical diseases or lesions, preferably caused by an infectious agent, in other words, preventing the disease or pathological condition from occurring in a subject (preferably a human), in particular, when said subject has a predisposition to suffer from the disease.

In the present invention, the term "vaginal or cervical diseases or lesions" refers to diseases that affect the external part of the female reproductive system, the vagina (vaginal diseases) and the cervix (cervical diseases), in other words, the area leading into the uterus, and cytological, histological and/or colposcopic alterations of the epithelium that are located on the surface of certain organs, such as the cervix, the vagina or the vulva and are characterised by mucosal thickenings that are opaque, white (leukoplakia), scaly and plaque-like which cause discomfort and itching (pruritus).

Examples of vaginal or cervical diseases include, but are not limited to: cervical cancer, vulvovaginitis, dermatophytosis, candidiasis, genital herpes, gonorrhoea, proctitis, infertility, cervicitis, pelvic inflammatory disease, ectopic pregnancy, acute or chronic pelvic pain and trichomoniasis.

In another preferred embodiment of the second medical use of the invention, the vaginal or cervical disease is selected from the list consisting of: cervical cancer, vulvovaginitis, dermatophytosis, candidiasis, genital herpes, gonorrhoea, proctitis, infertility, cervicitis, pelvic inflammatory disease, ectopic pregnancy, acute or chronic pelvic pain and trichomoniasis.

In another more preferred embodiment of the second medical use of the invention, the vaginal disease is cervical cancer.

Particularly, vaginal or cervical diseases or lesions may be caused by an infectious agent.

Thus, another preferred embodiment of the second medical use relates to the extract of *Hericium erinaceus* or the composition comprising it for use in the treatment and/or prevention of vaginal or cervical diseases and/or lesions caused by an infectious agent.

"Infectious agent" is the biological agent or microorganism capable of producing an infectious disease in a host, such as bacteria, helminths, fungi, prions, protozoa, viruses. Examples of infectious agents include, but are not limited to, human papillomavirus (HPV), *Candida albicans, Chlamydia trachomatis, Gardnerella vaginalis, Mobiluncus curtisii, Bacteroides fragilis, Mycoplasma genitalium, Neisseria gonorrhoeae, Trichomonas vaginalis,* herpes virus, *Staphylococcus aureus, Staphylococcus epidermis, Escherichia coli, Alpha streptococus, Gardnerella vaginalis, Enterobacter sakazakii, Proteus mirabilis, Enterobacter cloacae, and Klebsiella pneumoniae.*

In a more preferred embodiment of the second medical use of the invention, the infectious agent is selected from the list consisting of: human papillomavirus (HPV), *Candida albicans, Chlamydia trachomatis, Gardnerella vaginalis, Mobiluncus curtisii, Bacteroides fragilis, Mycoplasma genitalium, Neisseria gonorrhoeae, Trichomonas vaginalis,* herpes virus, *Staphylococcus aureus, Staphylococcus epidermis, Escherichia coli, Alpha streptococus, Gardenerella vaginalis, Enterobacter sakazakii, Proteus mirabilis, Enterobacter cloacae and Klebsiella pneumoniae.*

In another preferred embodiment of the second use of the invention, the infectious agent is the human papillomavirus (HPV).

In another more preferred embodiment of the second medical use of the invention, the vaginal disease is cervical cancer caused by the human papillomavirus (HPV).

Another even more preferred embodiment of the second medical use of the invention relates to the extract of *Hericium erinaceus* or the composition comprising it for use for the prevention and/or treatment of vaginal and/or cervical epithelial lesions caused by HPV.

In another preferred embodiment of the medical uses of the invention, the extract or the composition is administered orally, topical vaginally, parenterally, rectally and/or cervically.

Particularly, the inventors have discovered a synergistic effect in the simultaneous oral and topical vaginal administration of the extract of *Hericium erinaceus,* both the extract obtained from the mycelium and the extract obtained from the carpophore, or a composition comprising it in the treatment and/or prevention of vaginal or cervical diseases and/or lesions.

Thus, in another more preferred embodiment of the medical uses of the invention, the extract or the composition is administered simultaneously orally and topical vaginally.

The present invention also relates to the non-therapeutic use of the extract or of the composition of the invention, such as food or cosmetic uses.

Thus, another aspect of the invention relates to the use of the extract or of the composition of the invention for the preparation of human food products, preferably wherein the food product is a food, a nutritional supplement, a probiotic or a prebiotic. Said terms have been described in previous aspects of the invention and equally apply to the present inventive aspect.

Another aspect of the invention relates to the method for obtaining the carpophore extract of *Hericium erinaceus* (the extract of the invention), hereinafter "the method of the invention", comprising the following steps:
(i) mixing the entire carpophore or portions of carpophore of *Hericium erinaceus,* preferably smaller than 5 cm, with an aqueous solution and/or alcohol, preferably in a solid:liquid ratio of 1:5 to 1:40,
(ii) subjecting the heterogeneous mixture obtained in (i) to a temperature between 20°C and 70°C for between 30 and 60 minutes, obtaining an infusion of the mixture obtained in (i),
(iii) percolating or filtering the infusion obtained in (ii), obtaining the carpophore extract in the liquid fraction or solution.

The method of the invention can obtain the extract of *Hericium erinaceus* from the entire carpophore or carpophore cut into small pieces or portions, preferably smaller than 5 centimeters (cm), preferably smaller than 4 cm, more preferably smaller than 3 cm or smaller than 2 cm.

In another particular embodiment of the method of the invention, the portions of carpophore are smaller than 1 cm.

The entire carpophore or the carpophore pieces are mixed with a solvent, preferably an aqueous solution (more preferably water), an alcohol (more preferably ethanol, methanol, butanol, 2-propanol or pentanol), propane, butane, ethyl acetate, carbon dioxide, acetone, nitrous oxide or a mixture thereof.

In another more particular embodiment of the method of the invention, the entire carpophore or the portions of carpophore are mixed with aqueous solution and alcohol, preferably water and/or alcohol.

In another even more preferred embodiment of the method of the invention, the entire carpophore or the portions of carpophore are mixed with water and/or ethanol, more preferably with a mixture of water and ethanol.

When a mixture of ethanol and water is used, the ethanol is preferably found in a ratio between 10% by volume and 60% by volume (where the water would be found in a ratio between 90% and 40% by volume, respectively), more preferably between 20% and 60% by volume (where the water would be found in a ratio between 80% and 40% by volume, respectively), even more preferably between 40% and 60% by volume (where the water would be found in a ratio between 60% and 40% by volume, respectively).

In another more particular embodiment, in the method of the invention, the entire carpophore or the portions of carpophore are mixed with water and ethanol in a ratio of 50% and 50% by volume, in other words, in a water:ethanol ratio of 1:1.

Particularly, in the method of the invention, the ratio of the portions of carpophore or of said entire carpophore (solid) in the solvent (liquid), in other words, the solid:liquid ratio, is from 1:5 to 1:40.

In another particular embodiment, the ratio of the entire carpophore or the portions of carpophore (solid) in the solvent (liquid) is from 1:10 to 1:30, preferably 1:20.

The time required for extraction is usually adapted to the starting fungal material, the extraction method, the extraction temperature, the ratio between the solvent and the fungal material and the like.

Once the mixture of the entire carpophore or the portions of the carpophore and the solvent has been obtained, said mixture is subjected to a temperature of between 20 and 70°C for between 30 and 60 minutes (step (ii)), obtaining an infusion of the mixture obtained.

In another more particular embodiment, the mixture of the entire carpophore or the portions of carpophore and the solvent is subjected to a temperature of between 30 and 60°C, preferably between 35 and 55°C, for between 35 and 55 minutes, preferably between 40 and 50 minutes.

In another more particular embodiment, the mixture of the entire carpophore or the portions of carpophore and the solvent is subjected to a temperature of 36, 37, 38, 39, 40, 41, 42, 43, 44, 45, 46, 47, 48, 49, 50, 51, 52, 53 or 54°C for 41, 42, 43, 44, 45, 46, 47, 48 or 49 minutes.

In another even more preferred embodiment, the mixture of the entire carpophore or the portions of carpophore and the solvent is subjected to a temperature of 50°C for 45 minutes.

Optionally, the extraction step may be repeated on the same solid under the same conditions described above, in other words, step (ii) may optionally be repeated at least once.

After extraction (step ii), the mixture of solvent and extracted compounds that forms after the solvent comes into contact with the fungal material is separated from the fungal material by means of solid-liquid filtration by methods such as percolation, obtaining the carpophore extract of the invention in the liquid fraction.

The term "percolation" has been previously described and equally applies to this inventive aspect, as well as all of its preferred embodiments.

The bioactive compounds present in the extract, mainly phenolic compounds, usually have a limited useful life against oxidation. Therefore, additional drying of the extract (in the liquid fraction) can be optionally applied to prolong its useful life. One of the methods that can be applied for drying bioactive extracts is spray drying.

Thus, in another more preferred embodiment, the method of the invention further comprises a step (iv) of spray drying the solution obtained in (iii) at a temperature between 135 and 195°C.

In another even more particular embodiment, the temperature of step (iv) is between 145 and 185°C, preferably between 165 and 175°C.

During spray drying, an encapsulating agent can be added, for example, maltodextrin.

Thus, in another more preferred embodiment, the method of the invention further comprises a step (v) of adding an encapsulating agent, preferably the encapsulating agent is found in a ratio of between 1% m/m and 50% m/m (where the extract would be found in a ratio between 99% and 50% m/m, respectively), more preferably between 20% and 40% m/m (where the extract would be found in a ratio between 80% and 60% m/m, respectively), even more preferably between 25% and 35% m/m (where the extract would be found in a ratio between 75% and 65% m/m, respectively).

In another more particular embodiment, the extract:encapsulating agent ratio is 71.4% m/m and 28.57% m/m, in other words, in an extract:encapsulating agent ratio of 4:14.

Thus, in another more preferred embodiment, the method of the invention further comprises a step (v) of adding an encapsulating agent, preferably in an extract:encapsulating agent ratio of 4:14.

The term "encapsulating agent" has been previously described and equally applies to this inventive aspect, as well as all of its preferred embodiments.

In another more preferred embodiment, the method for obtaining the extract of the invention further comprises a step (v) of adding an encapsulating agent, where the encapsulating agent is maltodextrin.

### EXAMPLES

Next, the invention will be illustrated by means of assays carried out by the inventors that show the effectiveness of the extract of the invention in the treatment of vaginal and/or cervical lesions or diseases.

### 1. Process for obtaining the extract of Hericium erinaceus

The method for preparing a fungal extract of *Hericium erinaceus* from carpophore cut to a size smaller than 1 cm comprises:
a) extracting a fungal material by percolation using water and 50% ethanol as a solvent with a solid:liquid ratio of 1:20, for 45 minutes at a temperature of 50°C. After extraction, the mixture of solvent and extracted compounds that forms after the solvent comes into contact with the fungal material is separated from the fungal material by means of solid-liquid filtration.
b) drying the extract by spray drying. During spray drying, moisture evaporates at a temperature between 135°C and 195°C. During spray drying, maltodextrin is added as an encapsulating agent in an extract:encapsulating agent ratio of 4:14.

The carpophore extract of *Hericium erinaceus* obtained contains 24.37% m/m of beta-glucans, 51.2 µg/g of GABA, 348.2 µg/g of ergosterol and 0.2% m/m of Hericenone C.

The mycelial or micellar extract of the present invention relates to the extract obtained by the following production method, comprising the following steps:
(a) culturing a strain of *Hericium erinaceus* under aerobic conditions for 4 to 6 days (both values included), at a temperature between 25°C and 28°C (both values included), and at a pH of 6.0, in a liquid culture medium comprising brown sugar, potato flour, carrot flour, rice protein, oatmeal and virgin olive oil, to obtain a fermented product, and
(b) extracting, filtering and/or concentrating the fermented product obtained in step (a) to obtain a mycelial extract. The extraction is carried out by applying pressure and temperature, at 1 bar and at 121°C, for 9 to 11 minutes. After extraction, filtration is carried out through a 190 µm filter and subsequently through a 22 µm filter to remove residual biomass.

### 2. Cytotoxicity assay of the fungal extract on the Hela 229 cell line (human cervical adenocarcinoma).

Hela 229 cells are a human cervical adenocarcinoma cell line. Three segments of the genome of subtype 18 of human papillomavirus 18 (HPV 18) are integrated into the genome of the HeLa cells. This integration causes truncation in the E2 gene, a negative regulator of the viral expression of E6 and E7, which allows for the transcriptional activation of oncogenes. Compounds that inhibit the growth of this human cervical adenocarcinoma cell line could be used in the prevention and/or treatment of vaginal and/or cervical epithelial lesions (re-epithelialisation) and/or the treatment or prevention of diseases caused by the HPV infectious agent, particularly cervical cancer.

### 2.1. Hela 229 cell line culture conditions

Cytotoxicity studies of different fungal extracts were carried out on the Hela 229 cell line (human cervical adenocarcinoma cells). These cells were cultivated in DMEM (Dulbecco's Modified Eagle Medium) cell culture medium supplemented with 10% FBS (foetal bovine serum) and 2 mM L-glutamine in an atmosphere of 95% air and 5% CO₂ at 37°C.

### 2.2. Cytotoxicity study

The inhibition of cell growth induced by the different fungal extracts was evaluated using a system based on MTT tetrazollium salts (3-[4,5-dimethylthiazole-2-yl]-2,5-diphenyltetrazolium bromide), and on its ability to be transformed into formazan. Metabolically active living cells are able to reduce MTT, with a yellow colour in solution, to formazan, an insoluble compound that precipitates in the form of violet crystals, thus demonstrating metabolic activity. Formazan absorbs at a wavelength of 550 to 570 nm.

Hela 229 cells were seeded in a sterile 96-well plate at a density of 4,000 cells/well and incubated for 4-6 hours in the aforementioned growth medium. Subsequently, the different tested extracts dissolved in H₂O or DMSO were added to the cells, depending on the solubility of the ingredient, maintaining the same ratio of solvent in each well. After 72 hours of incubation in an atmosphere of 95% air and 5% CO₂ at 37°C, 10 µl of MTT at a concentration of 5 mg/mL prepared in phosphate buffered saline PBS (0.136 M NaCl, 1.47mM KH₂PO₄, 8 mM NaH₂PO₄ and 2.68 mM KCl) were added to each well and the cell plate was incubated for another 4 hours.

Subsequently, 100 µl of 10% SDS prepared in 0.01 M HCl were added and the cell plate was incubated for 12-14 hours under the same experimental conditions to dissolve the formazan precipitates. Lastly, the absorbance of the cell plate at a wavelength of 595 nm was measured (Tecan M1000 Infinite Pro). The measurement is taken after 48 h of incubation and the absorbance range varies between 0.1 and 1.

In all the experiments, controls with H₂O or DMSO in the same ratio in which the fungal extracts were dissolved were included and the absorbances were evaluated.

The experiments were carried out in triplicate. The data were expressed as percentage growth inhibition. This percentage inhibition was calculated based on the formula: % inhibition = 100 - ((AO x 100) / AT) where "AO" is the absorbance observed in the wells with the fungal extracts under study and "AT" is the absorbance observed in the wells with H₂O or DMSO controls.

### 2.3. Results

With the carpophore extract of *Hericium erinaceus* at concentrations of 1 mg/mL, 0.1 mg/mL y 0.02 mg/ mL, a percentage inhibition of HeLa cells of 42%, 46% and 41%, respectively, is obtained.

With a commercial carpophore extract of *Lentinula edodes* at concentrations of 0.1 mg/mL and 0.01 mg/mL, a percentage inhibition of 11% and 34%, respectively, is obtained (Table 1). The carpophore extract of *Lentinula edodes* used is a hydroalcoholic extract obtained by percolation, using water and ethanol as a solvent in a 1:1 ratio, at a solid-liquid ratio of 1:20 for 3 hours at a temperature of 45°C. The composition of the carpophore extract of *Lentinula edodes* comprises 9.07% m/m of beta-glucans, 205.7 µg/g of GABA, 584.21 µg/g of ergosterol and 12.01 µg/g of lovastatin.

With a commercial carpophore extract of *Grifola frondosa* at concentrations of 0.1 mg/mL, a percentage inhibition of 39% is obtained (Table 1). The carpophore extract of *Grifola frondosa* used is a hydroalcoholic extract obtained by percolation, using water and ethanol as a solvent in a 1:1 ratio, at a solid-liquid ratio of 1:20 for 3 hours at a temperature of 45°C. The composition of the carpophore extract of *Grifola frondosa* comprises 33.23% m/m of beta-glucans, 285.55 µg/g of ergosterol and 10 µg/g of lectin.

With a commercial carpophore extract of *Ganoderma lucidum* at concentrations of 0.1 mg/mL and 0.02 mg/mL, a percentage inhibition of 2% and 15%, respectively, is obtained (Table 1). The carpophore extract of *Ganoderma lucidum* used is a hydroalcoholic extract obtained by percolation, using water and ethanol as a solvent in a 1:1 ratio, at a solid-liquid ratio of 1:20 for 3 hours at a temperature of 45°C. The composition of the carpophore extract of *Ganoderma lucidum* comprises 48.69% m/m of beta-glucans, 403.8 µg/g ergosterol and 0.28% m/m of ganoderic acid.

Furthermore, according to the results shown in Table 1, the inventors have shown that the extract of *Hericium erinaceus* at a concentration of 0.1 mg/mL has a much higher percentage inhibition in Hela 229 cells than an extract of *Coriolus versicolor.* With a carpophore extract of *Coriolus versicolor* at concentrations of 0.1 mg/mL and 0.01 mg/mL, a percentage inhibition of 20% and 23%, respectively, is obtained, while with the carpophore extract of *Hericium erinaceus* at concentrations of 0.1 mg/mL and 0.02 mg/mL, a percentage inhibition of HeLa cells of 46% and 41 %, respectively, is obtained (Table 1). The carpophore extract of *Coriolus versicolor* used is a hydroalcoholic extract obtained by percolation, using water and ethanol as a solvent in a 1:1 ratio, at a solid-liquid ratio of 1:20 for 3 hours at a temperature of 45°C. The composition of the carpophore extract of *Coriolus versicolor* comprises 66.75% m/m of beta-glucans, 610.29 µg/g of ergosterol and 12.69 µg/g of lovastatin.

These results indicate that with a carpophore extract of *Hericium erinaceus* a higher % inhibition of HeLa cells is obtained in relation to other fungal species (Table 1).

**Table 1. Activity of fungus extracts (% inhibition of HeLa cells) at different concentrations.**

| Compound | Concentration (mg/mL) | % cell inhibition of the HeLa cells |
|---|---|---|
| Carpophore extract of *Hericium erinaceus* | 1 | 42 |
| Carpophore extract of *Hericium erinaceus* | 0.1 | 46 |
| Carpophore extract of *Hericium erinaceus* | 0.02 | 41 |
| Carpophore extract of *Lentinula edodes* | 0.1 | 11 |
| Carpophore extract of *Lentinula edodes* | 0.01 | 34 |
| Carpophore extract of *Coriolus versicolor* | 0.1 | 20 |
| Carpophore extract of *Coriolus versicolor* | 0.01 | 23 |
| Carpophore extract of *Grifola frondosa* | 0.1 | 2 |
| Carpophore extract of *Grifola frondosa* | 0.02 | 15 |
| Carpophore extract of *Ganoderma lucidum* | 0.1 | 39 |

### 3. Comparative cytotoxicity assay between the mycelial extract and the extract obtained from the carpophore in the Hela 229 cell line (human cervical adenocarcinoma).

The same methodology is used as in cytotoxicity assay 2 of the fungal extract in the Hela 229 cell line.

The inventors have discovered that with the carpophore extract of *Hericium erinaceus* a surprisingly higher percentage of HeLa cell inhibition is obtained than when a mycelial extract of this fungus is used. The carpophore extract of *Hericium erinaceus* at a concentration of 0.02 mg/mL gives an inhibition of 41% and a mycelial extract of *Hericium erinaceus* (obtained after a previously described fermentation process) at a concentration of 0.01 mg/mL provides inhibition between 1 and 8%.

**Table 2. Activity of the carpophore extract of Hericium erinaceus and of the mycelial extract of Hericium erinaceus (% inhibition of HeLa cells) at different concentrations**

| Compound | Concentratio n (mg/mL) | % cell inhibition of the HeLa cells |
|---|---|---|
| Carpophore extract of *Hericium erinaceus* | 1 | 42 |
| Carpophore extract of *Hericium erinaceus* | 0.1 | 46 |
| Carpophore extract of *Hericium erinaceus* | 0.02 | 41 |
| Mycelial extract of *Hericium erinaceus* | 0.01 | 1 to 8 |

## Claims

1. An extract obtained from the carpophore of *Hericium erinaceus.*

2. The extract according to claim 1, comprising 24.37% m/m of beta-glucans, 51.2 µg/g of GABA, 348.2 µg/g of ergosterol and 0.2% m/m of Hericenone C.

3. A composition comprising the extract according to claim 1 or 2, preferably wherein the composition comprises the extract of *Hericium erinaceus* in a concentration between 0.5% and 25% m/m.

4. The composition according to claim 3, wherein the composition is a nutritional composition or a pharmaceutical composition, preferably wherein the pharmaceutical composition further comprises a pharmaceutically acceptable carrier and/or excipient or wherein the nutritional composition is a food or nutritional supplement.

5. The composition according to claim 3 or 4, wherein the composition is in liquid, gel or solid form and/or is formulated for oral, topical vaginal, parenteral, rectal or cervical administration.

6. The extract according to claim 1 or 2 or the pharmaceutical composition according to claim 4 or 5 for use as a medicament.

7. An extract obtained from the mycelium or from the carpophore of *Hericium erinaceus* or a composition comprising it for use in the treatment and/or prevention of vaginal or cervical diseases and/or lesions, preferably vaginal or cervical epithelial lesions.

8. The extract for use according to claim 7, wherein the extract is the extract according to claim 1 or 2 or the composition is the composition according to claims 3 to 5.

9. The extract or the composition for use according to any one of claims 6 to 8, wherein the composition is administered orally, topical vaginally, parenterally, rectally and/or cervically, preferably wherein the composition is administered simultaneously orally and topical vaginally.

10. The extract or the composition for use according to any one of claims 7 to 9, wherein the vaginal or cervical disease or lesion is selected from the list consisting of: cervical cancer, vulvovaginitis, dermatophytosis, candidiasis, genital herpes, gonorrhoea, proctitis, infertility, cervicitis, pelvic inflammatory disease, ectopic pregnancy, acute or chronic pelvic pain and trichomoniasis, preferably wherein the vaginal disease is cervical cancer.

11. The extract or the composition for use according to any one of claims 7 to 10, wherein the vaginal or cervical disease or lesion is caused by an infectious agent, preferably wherein the infectious agent is selected from the list consisting of: human papillomavirus (HPV), *Candida albicans, Chlamydia trachomatis, Gardnerella vaginalis, Mobiluncus curtisii, Bacteroides fragilis, Mycoplasma genitalium, Neisseria gonorrhoeae, Trichomonas vaginalis,* herpes virus, *Staphylococcus aureus, Staphylococcus epidermis, Escherichia coli, Alpha streptococus, Gardnerella vaginalis, Enterobacter sakazakii, Proteus mirabilis, Enterobacter cloacae and Klebsiella pneumoniae,* preferably the human papillomavirus (HPV).

12. A method for obtaining the extract according to claim 1, comprising the following steps:
(i) mixing the entire carpophore or portions of carpophore of *Hericium erinaceus,* preferably smaller than 5 cm, with an aqueous solution and/or alcohol, preferably in a solid:liquid ratio of 1:5 to 1:40,
(ii) subjecting the mixture obtained in (i) to a temperature between 20°C and 70°C, preferably 50°C, for between 30 and 60 minutes, preferably 45 minutes, obtaining an infusion of the mixture obtained in (i),
(iii) percolating or filtering the infusion obtained in (ii), obtaining the carpophore extract in the liquid fraction or solution.

13. The method according to claim 12, further comprising a step (iv) of spray drying the solution obtained in (iii) at a temperature between 135 and 195°C, preferably between 165 and 175°C.

14. The method according to claim 12 or 13, wherein the method further comprises a step (v) of adding an encapsulating agent, preferably in an extract:encapsulating agent ratio of 4:14, more preferably wherein the encapsulating agent is maltodextrin.

15. An extract obtained by the method according to any one of claims 12 to 14.
